# EUROPEAN PATENT SPECIFICATION

(11) **EP 0 941 099 B1**
(45) Date of publication and mention of the grant of the patent: **17.09.2003**
(21) Application number: 97912761.0
(22) Date of filing: 21.10.1997
(51) Int. Cl.: A61K 31/70, A61K 31/7084, A61P 11/10, A61P 11/12

(54) **TREATMENT OF BRONCHITIS WITH DIURIDINE TETRAPHOSPHATE**
BEHANDLUNG VON BRONCHITIS MIT DIURIDINTRETRAPHOSPHAT
TRAITEMENT DE LA BRONCHITE AVEC DIURIDINE TETRAPHOSPHATE

(30) Priority: 07.11.1996 US 744367
(43) Date of publication of application: 15.09.1999
(73) Proprietor: Inspire Pharmaceuticals, Inc., Durham, NC 27703 (US)
(72) Inventor: SHAFFER, Christy, L., Chapel Hill, NC 27514 (US); BOUCHER, Richard, C., Chapel Hill, NC 27514 (US); RIDEOUT, Janet, L., Raleigh, NC 27607 (US); JACOBUS, Karla, M., Cary, NC 27511 (US)
(74) Representative: Schüssler, Andrea, Dr.
(86) International application number: PCT/US97/18766
(87) International publication number: WO 98/019685

(56) References cited:
- WO-A-92/11016
- WO-A-94/08593
- WO-A-96/40059
- US-A- 5 292 498
- A.T. BASSIN ET AL.: "Prevention of ventilator-associated pneumonia." CLIN. CHEST MED., vol. 16, no. 1, 1995, pages 195-208, XP002046472
- R.E. WOOD ET AL.: "Recent advances in aerosol therapy." J. AEROSOL MED., vol. 7, no. 1, 1994, pages 1-11, XP002046473
- K.N. OLIVER ET AL: "Acute safety and effects on mucociliary clearance of aerosolized uridine 5'-triphosphate+/- amiloride in normal human adults." AM. J. RESPIR. CRIT. CARE MED., vol. 154, no. 1, 1996, pages 217-223, XP002056055

## Description

### Technical Field

This invention relates to the use of a diuridine tetraphosphate for the preparation of a pharmaceutical composition for removing retained mucous secretions from the bronchi, bronchioles and small terminal airways of a subject.

### Background of the Invention

Chronic bronchitis (CB) is excessive production of mucus in the bronchi accompanied by a recurrent cough that persists for at least three months of the year during at least two successive years. CB is the major non-asthmatic disease of the lung. This condition affects approximately 14 million Americans and is a major cause of death in the United States. Many different factors initiate CB, including cigarette smoking, environmental pollution, chronic infections and various genetic abnormalities. Of these factors, cigarette smoking is the most prevalent. Pathological changes in the lung consist of: (1) hypertrophy and hyperplasia of mucus-secreting glands in the bronchi, (2) increase in goblet cells, (3) disappearance or damage of cilia, and (4) chronic inflammatory changes and narrowing of small airways. Often, a bacterial or viral infection is present. Excess amounts of mucus are found in the airways and sometimes may occlude small bronchioles. Eventually, there may be scarring of the bronchial wall. Coughing is stimulated by retained mucus which cannot be adequately removed due to decreased cilia and lessened mucociliary clearance (K. Svartengen, et al., *Exp. Lung Res.* 22, 555-76 (1996)). It is important that bronchitis patients clear retained mucus through coughing, however, often coughing is ineffective in adequately removing these secretions because the bronchitis patient cannot inspire deeply enough to cause air to flow distal to retained secretions.

Current treatments for chronic bronchitis include antibiotic therapy, bronchodilators, anti-inflammatory agents and chest physiotherapy. These treatments are often palliative in nature rather than effective in treating and/or preventing the progression of this disease. While antibiotics are effective in treating exacerbations of bronchitis due to bacterial infections, the disadvantage of antibiotic therapy is that the patient may develop antibiotic resistance. There is increasing evidence that chronic bronchitis is caused by viral infections. The disadvantage of bronchodilators is that they sometimes have adverse cardiovacsular side effects. As for anti-inflammatory agents, there is some controversy as to which stage in the progression of chronic bronchitis inflammation plays a role. None of these treatments have been successful in enhancing clearance of retained mucous secretions.

It is now known that nucleoside phosphates such as uridine 5'-triphosphate (UTP) and its analogs modulate components of the mucociliary clearance system . UTP has been shown to increase Cl⁻ secretion, and hence water secretion, from airway epithelial cells in vitro (S. Mason, et al., *Br. J. Pharmacol*. 103, 1649-56 (1991); see also, U.S. Patent No. 5,292,498 to R. Boucher (applicant intends the disclosure of this and all other patent references and publications cited herein be incorporated herein by reference). UTP has also been shown to increase cilia beat frequency in human airway epithelial cells in vitro (D. Drutz, et al., *Drug Dev. Res.* 37(3), 185 (1996)). UTP and other nucleotides have been shown to stimulate the release of surfactant phopholipids from type II alveolar cells (S. Rooney, et la., *Progr. Respir. Res.* 27, 84-91 (1994); L. Gobran, et al., Am J. Phsiol. 267, L625-33 (1994)). These effects have been shown to be mediated through a P2 receptor. The applicants believe that the release of surfactant or surface active molecules, caused as part of the treatment with UTP and related compounds, will improve compliance of the small airways and consequently improve gas exchange. Clinically, UTP has been shown to increase mucociliary lung clearance 2.5-fold in normal volunteers without any significant side-effects (K. Olivier, et al., *Am J. Respir. Care Med.* 154, 217-23 (1996)). UTP has also been shown to significantly improve cough clearance in primary ciliary dyskinesia (PCD) patients relative to vehicle (saline) (P. Noone, et al., *Amer. J. Respir. And Crit. Care Med*. 153(4), A530 (1996)). Also, in a subset of these PCD patients, the rate of sputum expectoration appeared to be increased with inhalation of UTP versus saline (unpublished data of applicants). Additionally, a French biotechnology company, Laboratories Synthelabo, has developed a pharmaceutical composition for treating nasal mucous fluid congestion under the trademark name rhinATP™ which uses adenosine triphosphate (ATP) as the active compound.

Further, WO 94/08593 discloses a method of hydrating mucous secretions in the lungs of a subject by administering UTP, and WO 96/40059 discloses the use of UTP for the preparation of a medicament for hydrating mucous secretions of a subject in need of such treatment. However, neither WO 94/08593 nor WO 96/40059 disclose administering a diuridane tetraphosphate.

Because of UTP's demonstrated ability to increase hydration of mucous secretions, increase cilia beat frequency and improve mucociliary whole lung clearance of retained secretions, applicants were motivated to investigate whether UTP, its analogs and other purinergic receptor agonists could effectively treat acute and chronic bronchitis as defined herein.

### SUMMARY OF THE INVENTION

A method of treating bronchitis in a subject in need of such treatment is disclosed. Bronchitis is defined to include chronic bronchitis, acute bronchitis and bronchiectasis. Cough clearance is defined as induction of lung mucus clearance by cough. The treating method comprises administering by inhalation an aerosol suspension of respirable particles to the bronchi, bronchioles and terminal small airways of the subject, the particles selected from the group consisting of general Formula II, i.e., P¹P⁴-di(uridine-5') tetraphosphate [U₂P₄] and its analogs, with the particles of Formula II, administered in an amount effective to hydrate retained bronchial mucous secretions and increase cilia beat frequency in the bronchi, bronchioles and terminal small airways of the subject, where by the retained mucous secretions are more easily transported from the bronchi, bronchioles and small terminal airways via mucociliary action.

UTP and its analogs are depicted in general Formula I: 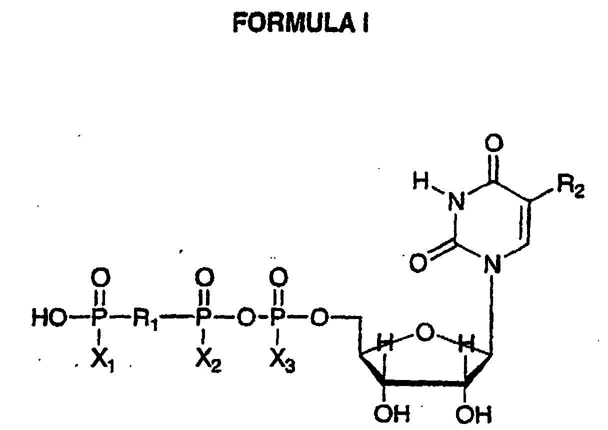 wherein:
X₁, X₂, and X₃ are each independently either O⁻ or S⁻. Preferably, X₂ and X₃ are O⁻.
R₁ is O, imido, methylene, or dihalomethylene (e.g., dichloromethylene, difluoromethylene). Preferably, R₁ is oxygen or difluoromethylene.
R₂ is H or Br. Preferably, R₂ is H. Particularly preferred compounds of Formula I are uridine 5'-triphosphate [UTP] and uridine 5'-O-(3-thiotriphosphate) [UTPγS].

A dinucleotide of to be used according to the present invention is depicted by the general Formula II: 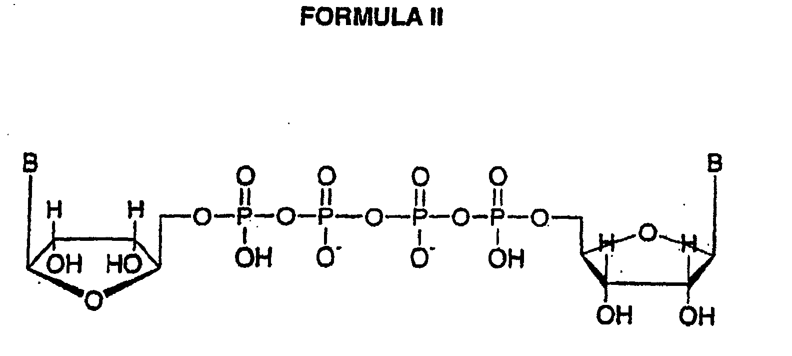 wherein:
B is uracil attached as shown in Formulae I and II.

For simplicity, Formula II herein illustrates the active compounds in the naturally occuring D-configuration, but the present invention also encompasses compounds in the L-configuration, and mixtures of compounds in the D- and L- configurations, unless otherwise specified. The naturally occuring D-configuration is preferred.

The present invention is directed to the use of a compound of Formula II, for the manufacture of a medicament for carrying out a therapeutic method of treatment as given above.

Used in the present invention is a pharmaceutical composition comprising compound of Formula II, in a pharmaceutical carrier in an amount effective to hydrate mucous secretions in the bronchi, bronchioles and small terminal airways; increase cilia beat frequency in the bronchi, bronchioles and small terminal airways; and enhance clearance of retained mucous secretions in the bronchi, bronchioles and small terminal airways.

### DETAILED DESCRIPTTON OF THE INVENTION

The present invention may be used to remove mucous secretions retained in the mainstem bronchi and small airways of a subject for any reason, including (but not limited to) retention of secretions arising from airway diseases such as acute or chronic bronchitis, bronchiectasis, asthma and emphysema. The compound uridine triphosphate was identified as a potent agonist of the P2Y₂ purinergic receptor in human airway epithelial preparations. The novel feature of uridine triphosphate as compared to other treatments for bronchitis, such as antibiotics, bronchodilators and anti-inflammatory agents is that this compound promotes hydration, stimulation of mucociliary and/or cough to enhance the removal of retained bronchial secretions.

The present invention is useful primarily in the treatment of human subjects, but may also be employed for the treatment of other mammalian subjects, such as dogs and cats, for veterinary purposes.

The term "uridine triphosphate" as used herein, include the pharmaceutically acceptable salts thereof, such as (but not limited to) an alkali metal salt such as sodium or potassium; an alkaline earth metal salt such as mangesium or calcium; or an ammonium or tetraalkyl ammonium salt, i.e., NH₄⁺ (wherein X is C₁₋₄ alkyl). Pharmaceutically acceptable salts are those that retain the desired biological activity of the parent compound and do not impart undesired toxicological effects.

The active compounds disclosed herein may be administered to the lungs of a patient by any suitable means, but are preferably administered by administering an aerosol suspension of respirable particles comprised of the active compound, which the subject inhales. The respirable particles may liquid or solid. The quantity of the active compound included may be an amount sufficient to achieve dissolved concentrations of the active compound on the bronchial surfaces of the subject of from about 10⁻⁷ to about 10⁻¹ Moles/liter, and more preferably from about 10⁻⁶ to about 5 x 10⁻² Moles/liter.

Depending upon the solubility of the particular formulation of active compound administered, the daily dose to promote secretion drainage may be divided among one or several unit dose administrations. The total daily dose for UTP may range from about 6 to 720 milligrams of respirable uridine triphosphate for a human subject, depending upon the age and condition of the subject. A currently preferred unit dose for UTP is about 2 to 100 milligrams of respirable urdine triphosphate particles given at a regimen of three to four administrations per day.

The liquid or solid particular uridine triphosphate prepared for practicing the present invention should include particles of respirable size: that is, particles of a size sufficiently small to pass through the mouth and larynx upon inhalation and into the bronchi, bronchioles and terminal small airways of the lungs. In general, particles ranging from about 1 to 5 microns in size are considered respirable.

Compounds of Formula II can be made in accordance with known procedures, or variations thereof which will be described by: E. Papaport, et al., *Proc. Natl. Acad. Sci.* USA, 72, 838-42 (1981); and K. Ng and L.E. Orgel, *Nucleic Acids Res.* 15(8), 3572-80 (1977).

In the manufacture of a formulation according to the invention, active agents or the physiologically acceptable salts thereof are typically admixed with, inter alia, an acceptable carrier. The carrier must be acceptable in that it is compatible with any other ingredients in the formulation and must not be deleterious to the patient. The carrier may be a solid or a liquid, or both. The formulations of the invention may incorporate one or more active compounds, and these formulations may be prepared by any of the well-known techniques of pharmacy consisting essentially of admixing the components.

Aerosols of liquid particles comprising the active compound may be produced by any suitable means, such as with a pressure-driven aerosol nebuilizer or an ultrasonic nebulizer. See, e.g., U.S. Patent No. 4,501,729. Nebulizers are commercially available devices which transform solutions or suspensions of the active ingredient into a therapeutic aerosol mist either by means of acceleration of compressed gas, typically air or oxygen, through a narrow venturi orifice or by means of ultrasonic agitation. Suitable formulations for use in nebulizers consist of the active incredient in a liquid carrier, the active ingredient comprising up to 40% w/w of the formulation, but preferably less than 20% w/w. The carrier is typically water (and most preferably sterile, pyrogen-free water) or a dilute aqueous alcoholic solution, preferably made isotonic with body fluids by the addition of, for example, sodium chloride. optional additives include preservatives if the formulation is not made sterile, for example, methyl hydroxybenzoate, antioxidants, flavoring agents, volatile oils, buffering agents and surfactants.

Aerosols of solid particles comprising the active compound may likewise be produced with any solid particulate medicament aerosol generator. Aerosol generators for administering solid particulate medicaments to a subject produce particles which are respirable, as explained above, and generate a volume of aerosol containing a predetermined metered dose of a medicament at a rate suitable for human administration. One illustrative type of solid particulate aerosol generator is an insufflator. Suitable formulations for administration by insufflation include finely comminuted powders which may be delivered by means of an insufflator or taken into the nasal cavity in the manner of a snuff. In the insufflator, the powder (e.g., a metered dose thereof effective to carry out the treatments described herein) is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the active ingredient or of a powder blend comprising the active ingredient, a suitable powder diluent, such as lactose, and an optional surfactant. The active ingredient typically comprises from 0.1 to 0.01 w/w of the formulation. A second type of illustrative aerosol generator comprises a metered dose inhaler. Metered dose inhalers are pressurized aerosol dispensers, typically containing a suspension or solution formulation of the active ingredient in a liquefied propellant. During use these devices discharge a metered volume, typically from 10 to 150 µl, to produce a fine particle spray containing the active ingredient. Suitable propellants include non-chlorofluorocarbon propellants as well as certain chlorofluorocarbon compounds, for example, dichlorodifluoromethane, trichlorofluoromethane, dichlorotetrafluoroethane and mixtures thereof. The formulation may additionally contain one or more cosolvents, for example, ethanol, surfactants, such as oleic acid or sorbitan trioleate, antioxidants and suitable flavoring agents.

Compositions containing respirable dry particles of micronized uridine triphosphate may be prepared by grinding the dry uridine triphosphate with a mortar and pestle, and then passing the micronized composition through a 400 mesh screen to break up or separate out large agglomerates. In dry powder delivery, the UTP may be formulated alone or in combination with diluent or carrier, such as sugars where the compounds may be intimately incorporated in the matrix through glassification or simply admixed with the carrier (i.e., lactose. sucrose, trehalose, mannitol) or other acceptable excipients for lung or airway delivery. The dry powder may be obtained by methods known in the art, such as spray-drying, milling, freeze-drying, etc.

The aerosol, whether formed from solid or liquid particles, may be produced by the aerosol generator at a rate of from about 10 to 150 liters per minute, more preferable from about 30 to 150 liters per minute, and most preferable about 60 liters per minute. Aerosols containing greater amounts of medicament may be administered more rapidly.

The particulate uridine triphosphate composition may optionally contain a dispersant which serves to facilitate the formation of an aerosol. A suitable dispersant is lactose, which may be blended with the uridine triphosphate in any suitable ratio (e.g., a 1 to 1 ratio by weight).

Another means of administering the active compound to the bronchi of the subject would involve administering a liquid/liquid suspension (either a nasal spray of respirable particles which the subject inhales, or nasal drops of a liquid formulation, or eye drops of a liquid formulation) comprised of the active compound. Liquid pharmaceutical compositions of the active compound for producing a nasal spray or nasal or eye drops may be prepared by combining the active compound with a suitable vehicle, such as sterile pyrogen free water or sterile saline by techniques known to those skilled in the art.

Another means of administering the active compound would involve oral administration, in which pharmaceutical compositions containing compounds of Formula II, are in the form of tablets, lozenges, aqueous or oily suspensions, dispersible powders or granules, emulsion, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with nontoxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed. Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Another means of administering the active compound to the bronchi of the subject would involve a suppository form of the active compound, such that a therapeutically effective amount of the compound reaches the lung via systemic absorption and circulation.

Another means of administering the active compound would involve direct intra-operative instillation of a gel, cream, or liquid suspension form of a therapeutically effective amount of the active compound. Such intra-operative instillation could take place during bronchoscopy, thoracotomy or during surgery to remove non-functioning, hyper-inflated sections of the lung, as is sometimes required in advanced stages of bronchitis, bronchiectasis or emphysema

Those having skill in the art will recognize that the starting materials may be varied and additional steps employed to produce compounds encompassed by the present invention, as demonstrated by the following examples. In some cases protection of certain reactive functionalities may be necessary to achieve some of the above transformations. In general the need for such protecting groups will be apparent to those skilled in the art of organic synthesis as well as the conditions necessary to attach and remove such groups.

The invention is illustrated further by the following examples which are not to be construed as limiting the invention in scope or spirit to the specific procedures described in it.

### EXPERIMENTAL

### Example 1

### Trachael Mucus Study in Sheep

The effects of UTP and U₂P₄ on trachael mucus velocity (TMV) were studied using the following procedures: The nasal passages of conscious adult ewes were anesthetized with a 2% lidocaine solution. After local anesthesia was produced, a modified endotracheal tube 7.5 mm was placed such that the cuff was just below the vocal cords (verified by fluoroscopy). Inspired air was warmed and humidified. The cuff of the endotracheal tube was inflated only during administration of the test compound to minimize possible impairment of TMV by the cuff. Test compounds were administered by nebulization in a volume of 4 mL over a period of 10-12 min.

TMV was measured by fluoroscopy. Ten to twenty radiopaque disks (Teflon• /bismuth trioxide; 1 mm diameter, 0.8 mm thich, weighing 1.8 mg) were introduced into the trachea through a modified suction catheter with a puff of compressed air (3-4 L/min). Velocities of the individual disks were recorded on videotape from a portable image intensifier unit. Individual disk velocities were calculated by measuring the distance traveled by each disk during a 1 min observation period. Values reported are the means of the individual disk velocities. A collar was worn by the sheep which was used as a standard to correct for magnification errors inherent in the fluoroscope.

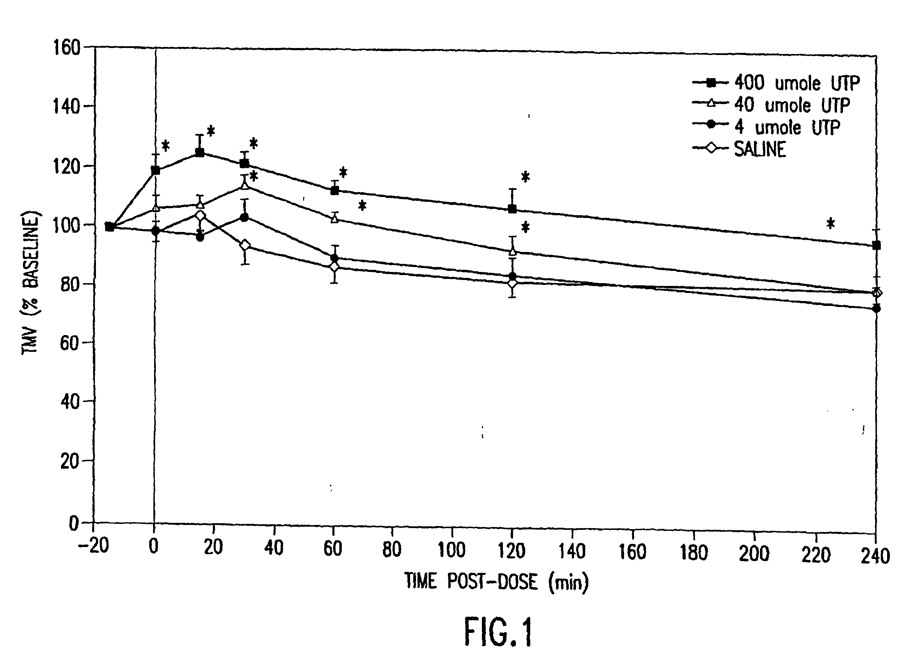

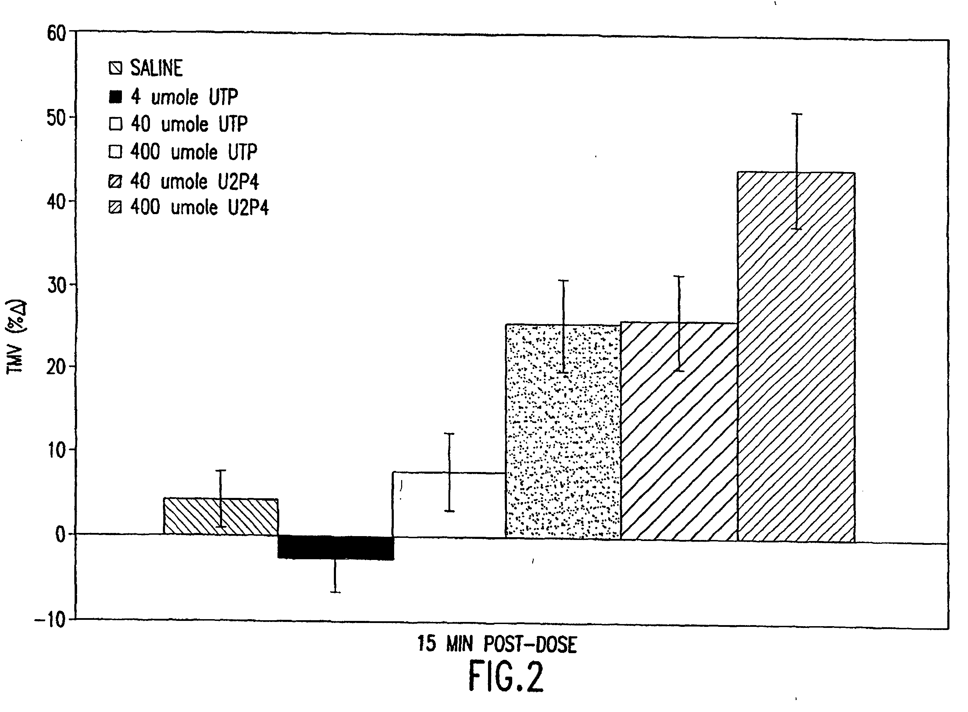

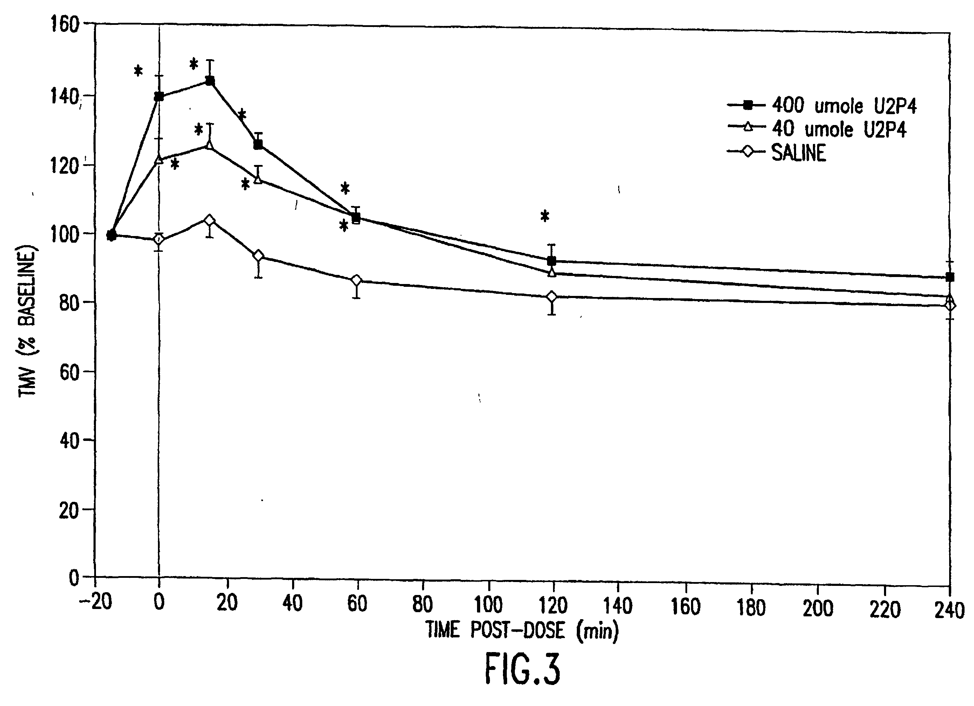

Both UTP and U₂P₄ produced significant dose-related effects on tracheal mucus velocity. The doses ranged from 4 to 400 µmole. Both compounds had their maximal effects at a dose of 400 µmole (4 ml of 10⁻¹M). UTP produced a maximal effect of 125 ± 7% of baseline (mean ± standard error, n = 6). U₂P₄ produced a maximal effect of 144 ± 9% of baseline (n = 6). Both compounds produced their maximal effects 15 min after administration. The highest dose of UTP produced significant effects on TMV up to 4 h after administration. The effects of U₂P₄ were significant out to 2 h after administration. Results are shown in Figures 1 - 3.

### Example 2

### Mucociliary Clearance Study in Sheep

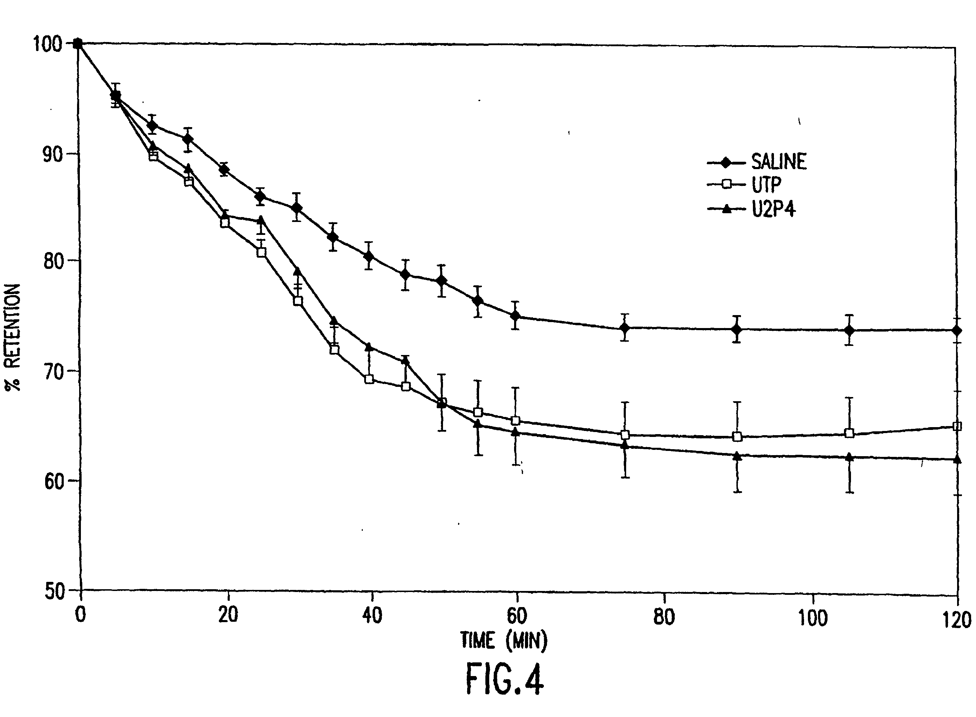

In this study healthy adult ewes were given ^{99m}Tc-labeled human serum albumin (^{99m}Tc-HSA) via a nebulized aerosol. The ^{99m}TC-HSA (20mCi) was administered over 5 min through a nasotracheal tube introduced under local anesthesia with 2% lidocaine. After administration of the ^{99m}Tc-HSA, the animals were given a test compound: either UTP or U₂P₄. Test compounds were administered by nebulization in a volume of 4 mL over a period of 10-12 min. The test compounds were given at a dose of 400 µmole. After the administration of the test compound, the animals were extubated. Clearance of the radiolabeled particles was monitored with a gamma camera. Measurements were made at 0, 5, 10, 15, 20, 25, 30, 35, 40, 45, 50, 55, 60, 76, 90, 105 and 120 min. Initial results (n = 7) have shown that both test compounds promote clearance of the radiolabeled particles (compared to the saline control). Results are shown in Figure 4.

The results of the studies in sheep on tracheal mucus velocity (TMV) and whole lung mucociliary clearance (WLC) demonstrated that UTP and U₂P₄ can enhance mucociliary clearance. UTP and U₂P₄ were able to produce an enhancement of TMV. These data strongly suggest that UTP and U₂P₄ enhance both TMV and mucociliary clearance, which is useful as shown in the clinical trials shown below (Example 4).

### Example 3 (Reference example)

### Treatment of a Human Subject with Mild Chronic Bronchitis

It has been demonstrated that two different doses of UTP given by inhalation (4ml of a liquid solution containing either 5 mg/ml or 25 mg/ml UTP) enhances mucociliary clearance over baseline (no inhaled treatment) and also over placebo, in a patient with mild chronic bronchitis. In this study, the patient inhaled the different doses of UTP and placebo on separate days. The procedure for measuring mucociliary clearance was accomplished by having the patient inhale, in the following order: technetium-labeled iron oxide particles and then either one of the two doses of UTP or placebo. After inhalation of the UTP or placebo, clearance of the radiolabel particles was measured by serial 2-minute gamma scintigraphy images for 2.5 hours. In order to establish baseline mucociliary clearance on the first day of the study, the patient inhaled only the radiolabel and then had the 2.5 hour gamma scintigraphy images performed. Safety data was collected by monitoring heart rate, ECG, blood pressure, oxyhemoglobin saturation by pulse oximetry prior to, during, and after inhalation for all dosing periods. During all phases of the study the patient in this study and the patients in the study described in Example 4 were monitored for any adverse reactions during each dosing period.

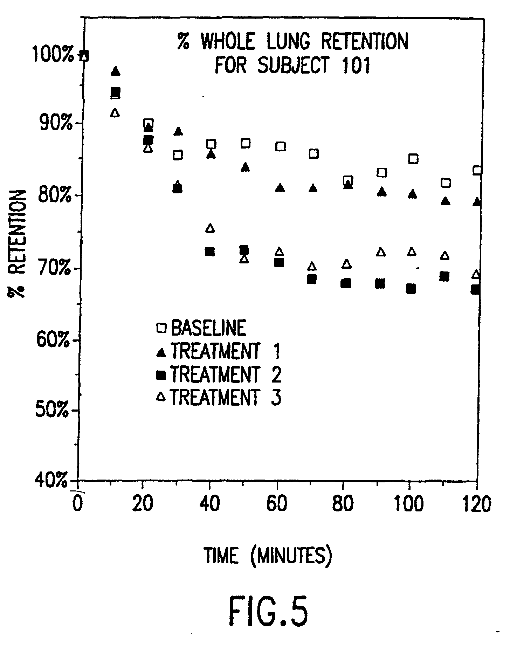

Figure 5 shows the Whole Lung Retention curves for baseline (no inhalation of UTP or placebo), both doses of UTP (5 mg/ml is labeled as "treatment 3"; 25 mg/ml is labeled as "treatment 2"), and placebo (labeled as "treatment 1") over the 2.5 hour time period. A decline in retention demonstrates a clearance of the secretions in the lungs carrying the radiolabel particles. In this case, both doses of UTP clearly enhance the clearance of secretions (mucociliary clearance) over placebo and baseline.

### Example 4 (Reference example)

### Clinical Study in Patients with Chronic Bronchitis

This single-center study, conducted at a major academic center in the U.S., was a randomized, double-blind, cross-over evaluation of escalating, single inhaled doses of UTP in 26 adult patients with chronic bronchitis (CB). Patients enrolled in this study had to meet the American Thoracic Society definition of chronic bronchitis (excessive mucous production over 3 months of the year, for at least 2 successive years). Patients were included that had mild to moderate airflow obstruction (forced expiratory volume over 1 second > 65% of predicted at study entry). The majority of patients in this study were smokers; 14 patients were females and 12 were males. Five successive groups of five subjects (an additional patient was added in one cohort due to a dropout) received a single dose of placebo and the appropriate dose of UTP (2.5, 5, 15, 25 and 45 mg/mL) in a randomized order. The dose of UTP and placebo were separated by at least 24 hours. The purpose of this study was to more carefully define the timecourse by which UTP enhances the expectoration of sputum in a patient with CB. This study also included evaluation of the numbers of specific cell types in the sputum (alveolar macrophages, ciliated epithelial cells, eosinophils, and neutrophils).

### - Treatment Assignments and Administration of Study Drug

Each subject was randomly assigned to receive either 1) a single inhaled dose of placebo (normal saline) or 2) one of five doses of UTP, over two separate days. For dosing, each dose consisted of 4mL of 2.5, 5, 15, 25 or 45 mg/mL solution or placebo (normal saline) administered using a jet nebulizer (Pari LC PLUS™) powered by a portable compressor set at 14 L/min. Inhalation of placebo or UTP took approximately 8-15 minutes.

### - Efficacy Results

The amount of sputum expectorated (weight in grams) was collected at baseline and at various time points post-dosing of UTP and placebo. The timepoints were: immediately to 5 minutes post-dosing, 6 minutes to 30 minutes post-dosing, and 31 minutes post-dosing to discharge (within several hours of post-dosing).

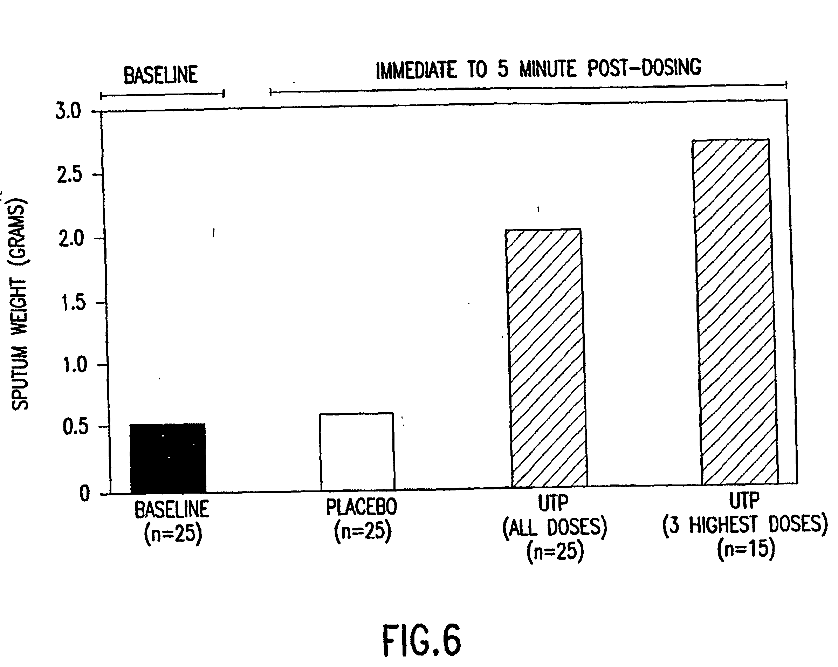

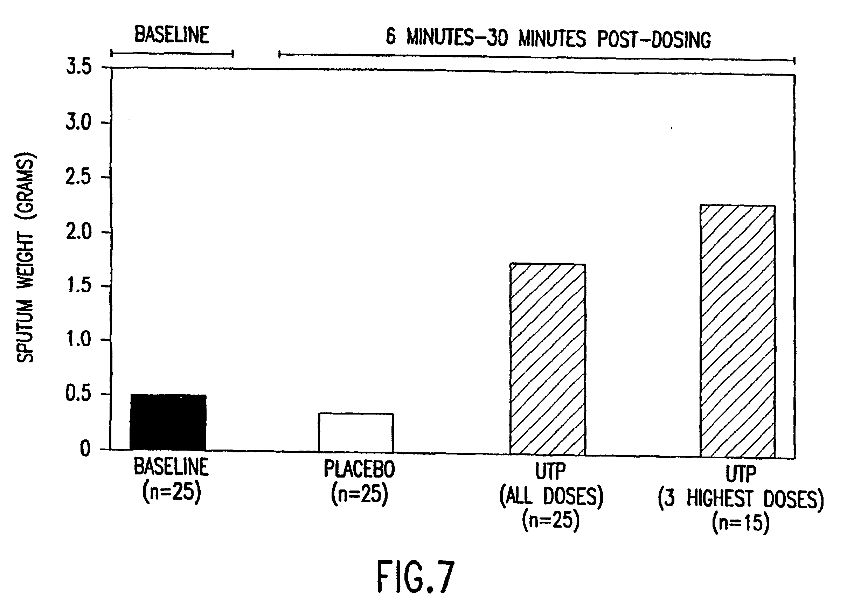

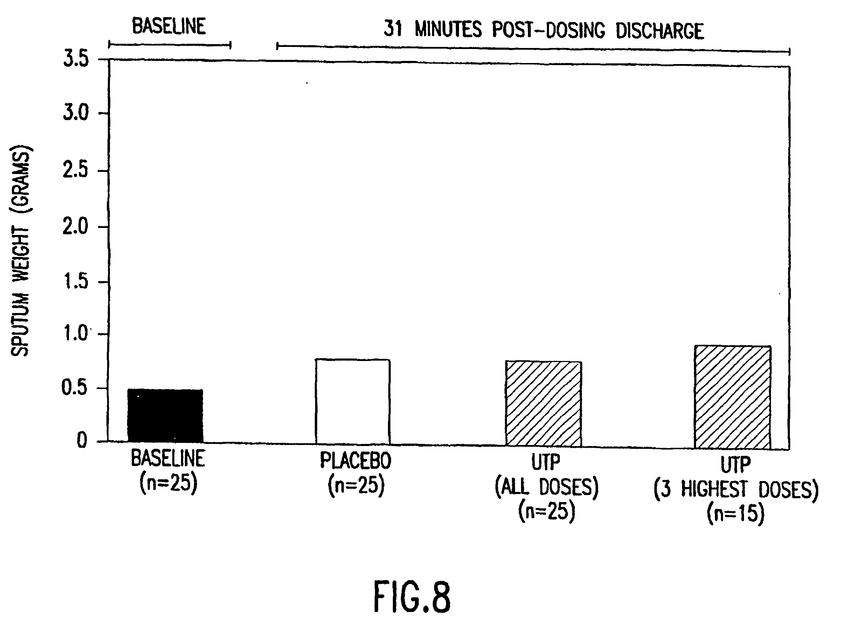

For the purposes of analysis, patients receiving placebo across all dose groups were combined (n=25); patients receiving UTP (all doses) were combined; and patients receiving the three highest dose levels (15, 25 and 45 mg/mL) [n=15] were combined. Figure 6 illustrates the effect of placebo versus UTP on the amount of sputum expectorated (weight in grams) at two time points: baseline (spontaneous expectoration) versus immediately to 5 minute post-dosing. UTP (all doses combined) significantly enhanced the amount of sputum expectorated compared to baseline and placebo (all doses combined). The effect of UTP was even more pronounced when comparing the three highest dose levels of UTP (n=15) to the placebo group. The ability of UTP to enhance the amount of sputum expectorated over placebo and baseline was also quite evident at the later timepoint of 6 - 30 minutes post-dosing, as shown in Figure 7. Figure 8 shows that at the 31 minute - discharge time period there was essentially no difference between the effect of UTP and placebo on the amount of sputum expectorated, indicating that the effect of UTP is manifest over a short time, consistent with previous studies in other patient populations.

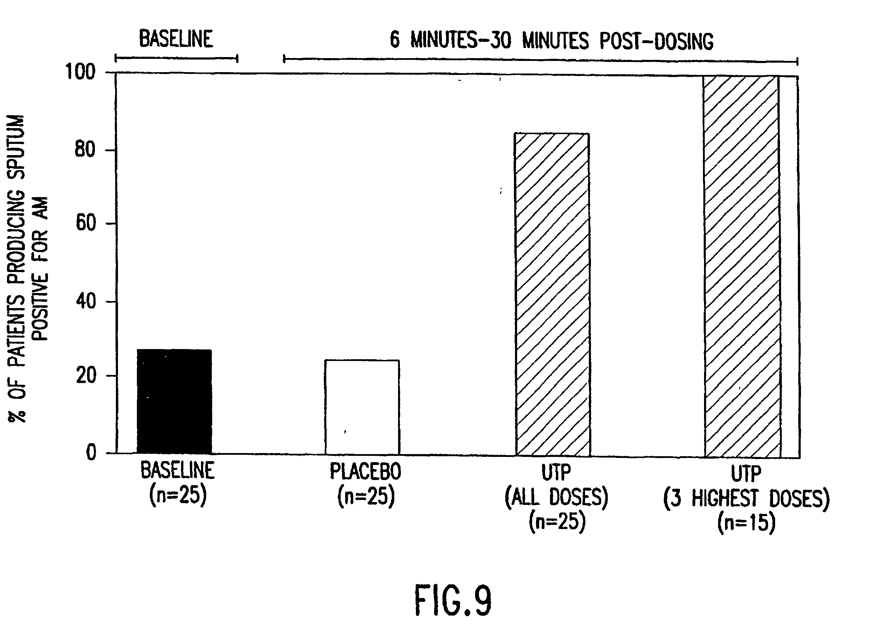

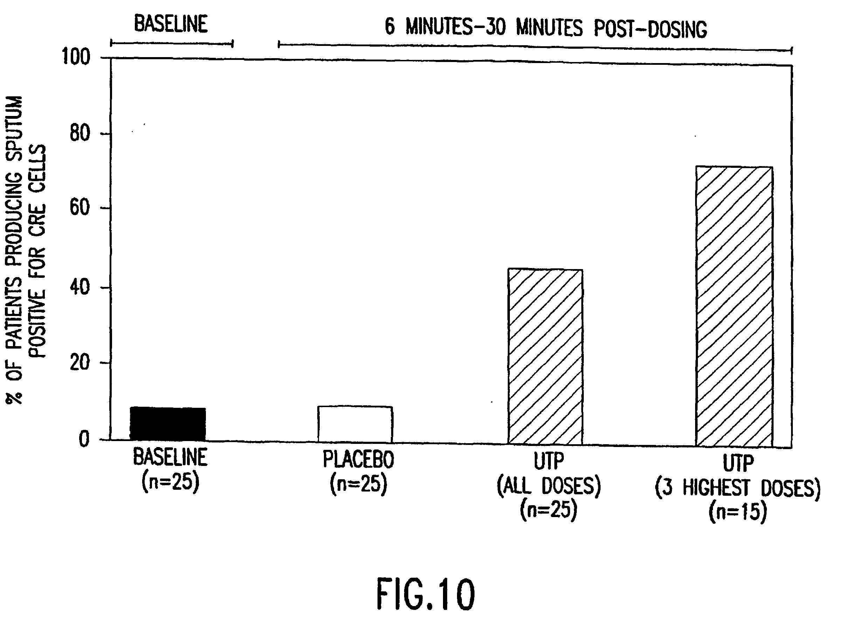

The cytology results from sputum samples collected at the 6-30 minute post-dosing time point are shown in Figures 9 (sputum containing alveolar macrophages) and Figure 10 (sputum containing respiratory ciliated epithelial cells). UTP (all doses combined) and UTP (three highest dose levels combined) significantly improved the percentage of patients producing a sputum sample containing alveolar macrophages (AM) compared to placebo (Figure 9). UTP (all doses combined) and UTP (three highest dose levels combined) also significantly improved the percentage of patients producing a sputum sample containing respiratory ciliated epithelial cells, when compared to placebo (Figure 10). It is noteworthy in Figure 10 that only 8% of the patients were able to produce a sample containing ciliated epithelial cells at baseline or after aerosolization of placebo; whereas, 73% of patients could produce such a sample in the UTP (3 highest dose level) group. These effects of UTP were also observed at the 0 to 5-minutes post-dosing timepoint for alveolar macrophages (UTP [all doses] = 32%; baseline/placebo = 8%/4%; UTP [3 highest doses] = 50%). The effects of UTP had returned to close to the baseline/placebo values at the 31-minute to discharge timeframe, consistent with the findings on sputum weight.

This study clearly demonstrates that aerosolized doses of UTP (particularly at the three highest dose levels of 15, 25 and 45 mg/mL) are more effective than placebo (normal saline) in enhancing the expectoration of sputum. Based on the types of cells present, it is clear that the sputum is produced from the deep lung, and is not simply secretions from the oropharynx. The effect of UTP to enhance the clearance of lung secretions (sputum) was manifest over a very short timeframe (within 30 minutes post-dosing), which is consistent with the effect seen in studies of UTP in normal subjects (including smokers) and in one adult patient with CB on whole lung mucociliary clearnace (Example 3).

In another study conducted in normal male smokers who did not have any lung obstruction (i.e., development of lung disease similar to chronic bronchitis from smoking), the subjects were given foramlated UTP by inhalation aerosol three times a day for three consecutive days. The study was a randomized, placebo-controlled, crossover design (each subject received both UTP and placebo for 3 days each, separated by one week; order of treatment was randomly determined). Enhanced sputum production (and therefore mucociliary clearance) after inhalation of UTP versus placebo occurred within the same timeframe as described above (data not known).

In summary, data (Examples 1-4) from several pre-clinical (animal) and clinical (human) studies demonstrate that inhaled UTP significantly enhances whole lung mucociliary clearance as measured by gamma scintigraphy studies and as shown by enhanced removal of retained lung secretions (sputum expectoration). Clearance and removal of these retained secretions prevents complications and improves the health of patients with chronic bronchitis.

The subject methods and compounds described herein provide a means for treatment of bronchitis. The use according to the present invention comprises administering to the airways of the subject an anlog of a uridine triphosphate such as uridine 5'-triphosphate (UTP), namely, U₂P₄, in an amount effective to hydrate mucous secretions, to promote mucociliary and/or cough clearance, or to stimulate ciliary beat frequency and the release of surfactant or surface active molecules to promote improved compliance and gas exchange in the lungs.

## Claims

1. Use of a compound of the following formula, or a pharmaceutically acceptable salt thereof, in a pharmaceutical carrier having an amount of said compound effective to hydrate bronchial mucous secretions or increase bronchial cilia beat frequency so that mucociliary clearance or cough clearance is enhanced: 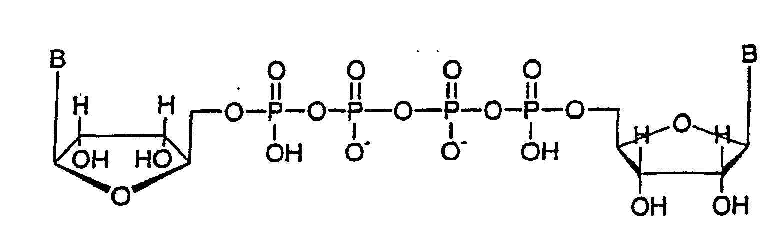 wherein
B is uracil 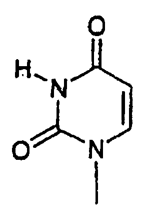 for preparing a pharmaceutical composition for use in a method of treating bronchitis in a subject in need of such treatment, wherein said compound is administered in an amount sufficient to achieve dissolved concentrations thereof on the surfaces of the bronchi, bronchioles and small terminal airways of said subject of from about 10⁻⁷ to about 10⁻¹ moles/liter.

2. The use according to claim 1, wherein said compound is delivered by administering a nebulized aerosol or suspension or solution of said compound to the nasopharyngeal airways of said subject, such that a therapeutically effective amount of said compound contacts the bronchi, bronchioles and small terminal airways of said subject either directly or by systemic absorption and circulation.

3. The use according to claim 1, wherein said compound is delivered by administering an oral form of said compound, such that a therapeutically effective amount of said compound contacts the bronchi, bronchioles and small terminal airways of said subject via systemic absorption and circulation.

4. The use according to claim 1, wherein said compound is delivered by administering a liquid/liquid suspension, including eye drops of said compound to the eyes, or nasal drops, powder or spray, of said compound to the nasopharyngeal airways of said subject, such that a therapeutically effective amount of said compound contacts the bronchi, bronchioles and small terminal airways subject either directly or via systemic absorption and circulation.

5. The use according to claim 1, wherein said compound is delivered by administering an injected form of said compound, such that a therapeutically effective amount of said compound contacts the bronchi, bronchioles and small terminal airways of said subject via systemic absorption and circulation.

6. The use according to claim 1, wherein said compound is delivered by administering a suppository form of said compound, such that a therapeutically effective amount of said compound contacts the bronchi, bronchioles and small terminal airways of said subject via systemic absorption and circulation.

7. The use according to claim 1, wherein said compound is delivered by administering an intra-operative instillation of a gel, cream, powder, foam, crystals or liquid suspension form of the active compound such that a therapeutically effective amount of said compound contacts the bronchi, bronchioles and small terminal airways of said subject via systemic absorption and circulation.

8. The use according to claim 1, wherein said compound is P¹, P⁴-di(uridine-5')tetraphosphate(U₂P₄)and pharmaceutically acceptable salts thereof.

## Patentansprüche

1. Verwendung einer Verbindung der folgenden Formel oder eines pharmazeutisch annehmbaren Salzes davon in einem pharmazeutischen Träger mit einer Menge an Verbindung, die dahingehend wirksam ist, dass sie bronchiale Schleimsekrete hydratisiert oder die bronchiale Zilienbewegungsfrequenz erhöht, um so die mukoziliare Freisetzung oder die Freisetzung durch Abhusten zu verstärken: 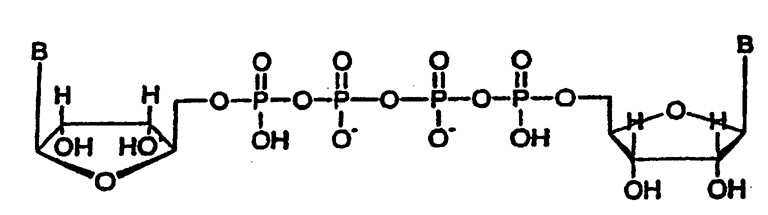 worin
B Uracil ist 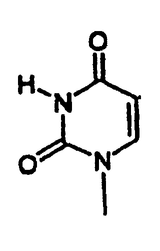 zum Herstellen einer pharmazeutischen Zusammensetzung zur Verwendung bei einem Verfahren zum Behandeln von Bronchitis bei einer Person, die einer solchen Behandlung bedarf, wobei die Verbindung in einer Menge verabreicht wird, die ausreicht, um aufgelöste Konzentrationen davon an den Oberflächen der Bronchien, Bronchiolen und kleinen terminalen Luftwege der Person von ungefähr 10⁻⁷ bis ungefähr 10⁻¹ mol/Liter zu erreichen.

2. Verwendung nach Anspruch 1, wobei die Verbindung durch Verabreichen eines vernebelten Aerosols oder Suspension oder Lösung der Verbindung an die nasopharyngealen Luftwege der Person zugeführt wird, so dass eine therapeutisch wirksame Menge an Verbindung die Bronchien, Bronchiolen und kleinen terminalen Luftwege der Person entweder direkt oder durch systemische Absorption und Zirkulation kontaktiert.

3. Verwendung nach Anspruch 1, wobei die Verbindung durch Verabreichen einer oralen Form der Verbindung zugeführt wird, so dass eine therapeutisch wirksame Menge an Verbindung die Bronchien, Bronchiolen und kleinen terminalen Luftwege der Person über systemische Absorption und Zirkulation kontaktiert.

4. Verwendung nach Anspruch 1, wobei die Verbindung durch Verabreichen einer Flüssig/Flüssig-Suspension, einschließlich Augentropfen der Verbindung an die Augen oder Nasentropfen, Pulver bzw. Puder oder Spray der Verbindung an die nasopharyngealen Luftwege der Person zugeführt wird, so dass eine therapeutisch wirksame Menge an Verbindung die Bronchien, Bronchiolen und kleinen terminalen Luftwege der Person entweder direkt oder über systemische Absorption und Zirkulation kontaktiert.

5. Verwendung nach Anspruch 1, wobei die Verbindung durch Verabreichen einer injizierten Form der Verbindung zugeführt wird, so dass eine therapeutisch wirksame Menge an Verbindung die Bronchien, Bronchiolen und kleinen terminalen Luftwege der Person über systemische Absorption und Zirkulation kontaktiert.

6. Verwendung nach Anspruch 1, wobei die Verbindung durch Verabreichen einer Suppositoriumsform der Verbindung zugeführt wird, so dass eine therapeutisch wirksame Menge an Verbindung die Bronchien, Bronchiolen und kleinen terminalen Luftwege der Person über systemische Absorption und Zirkulation kontaktiert.

7. Verwendung nach Anspruch 1, wobei die Verbindung durch Verabreichen einer intraoperativen Instillation eines Gels, einer Creme, eines Pulvers oder Puders, eines Schaums, von Kristallen oder einer Flüssigsuspensionsform der aktiven Verbindung zugeführt wird, so dass eine therapeutisch wirksame Menge an Verbindung die Bronchien, Bronchiolen und kleinen terminalen Luftwege der Person über systemische Absorption und Zirkulation kontaktiert.

8. Verwendung nach Anspruch 1, wobei die Verbindung P¹,P⁴-Di-(uridin-5')tetraphosphat (U₂P₄) und pharmazeutisch annehmbare Salze davon umfasst.

## Revendications

1. Utilisation d'un composé de formule suivante, ou d'un sel pharmaceutiquement acceptable de celui-ci, dans un support pharmaceutique ayant une quantité dudit composé efficace pour hydrater les sécrétions muqueuses bronchiques ou augmenter la fréquence des battements des cils bronchiques de telle sorte que la clairance mucociliaire ou la clairance de la toux est augmentée : 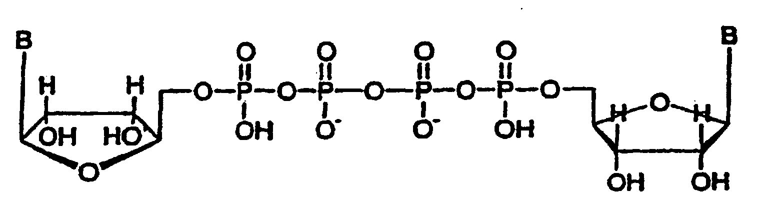 où
B est l'uracile 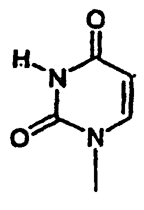 pour préparer une composition pharmaceutique destinée à être utilisée dans une méthode de traitement de la bronchite chez un sujet qui a besoin d'un tel traitement, où ledit composé est administré en une quantité suffisante pour obtenir des concentrations dissoutes de celui-ci sur les surfaces des bronches, des bronchioles et des petites voies aériennes terminales dudit sujet d'environ 10⁻⁷ à environ 10⁻¹ mole/litre.

2. Utilisation selon la revendication 1 où ledit composé est délivré par administration d'un aérosol nébulisé ou d'une suspension ou solution nébulisée dudit composé aux voies aériennes naso-pharyngiennes dudit sujet, de sorte qu'une quantité thérapeutiquement efficace dudit composé entre en contact avec les bronches, les bronchioles et les petites voies aériénnes terminales dudit sujet directement ou par absorption et circulation systémiques.

3. Utilisation selon la revendication 1 où ledit composé est délivré par administration d'une forme orale dudit composé, de sorte qu'une quantité thérapeutiquement efficace dudit composé entre en contact avec les bronches, les bronchioles et les petites voies aériennes terminales dudit sujet par absorption et circulation systémiques.

4. Utilisation selon la revendication 1 où ledit composé est délivré par administration d'une suspension liquide/liquide, incluant des gouttes oculaires dudit composé aux yeux, ou des gouttes nasales, une poudre nasale ou un spray nasal, dudit composé aux voies aériennes naso-pharyngiennes dudit sujet, de sorte qu'une quantité thérapeutiquement efficace dudit composé entre en contact avec les bronches, les bronchioles et les petites voies aériennes terminales dudit sujet directement ou par absorption et circulation systémiques.

5. Utilisation selon la revendication 1 où ledit composé est délivré par administration d'une forme injectée dudit composé, de sorte qu'une quantité thérapeutiquement efficace dudit composé entre en contact avec les bronches, les bronchioles et les petites voies aériennes terminales dudit sujet par absorption et circulation systémiques.

6. Utilisation selon la revendication 1 où ledit composé est délivré par administration d'une forme de suppositoire dudit composé, de sorte qu'une quantité thérapeutiquement efficace dudit composé entre en contact avec les bronches, les bronchioles et les petites voies aériennes terminales dudit sujet par absorption et circulation systémiques.

7. Utilisation selon la revendication 1 où ledit composé est délivré par administration d'une instillation intra-opératoire d'une forme de gel, de crème, de poudre, de mousse, de cristaux ou de suspension liquide du composé actif, de sorte qu'une quantité thérapeutiquement efficace dudit composé entre en contact avec les bronches, les bronchioles et les petites voies aériennes terminales dudit sujet par absorption et circulation systémiques.

8. Utilisation selon la revendication 1 où ledit composé est le P¹,P⁴-di(uridine-5')tétraphosphate (U₂P₄) et ses sels pharmaceutiquement acceptables.
